# EUROPEAN PATENT SPECIFICATION

(11) **EP 1 390 083 B1**
(45) Date of publication and mention of the grant of the patent: **12.04.2006**
(21) Application number: 02766692.4
(22) Date of filing: 29.04.2002
(51) Int. Cl.: A61L 9/01, A61L 9/013

(54) **MULTI-FUNCTIONAL MATERIAL EMITTING FAR-INFRARED RAY IN AQUEOUS PHASE AND THE USE THEREOF**
STRAHLEN IM WEITEN INFRAROTBEREICH AUSSENDENDES MULTIFUNKTIONALES MATERIAL IN WÄSSRIGER PHASE UND SEINE VERWENDUNG
MATIERE A FONCTIONS MULTIPLES EMETTANT UN RAYONNEMENT INFRAROUGE LOINTAIN DANS UNE PHASE AQUEUSE ET SON UTILISATION

(30) Priority: 30.04.2001 KR 2001023305
(43) Date of publication of application: 25.02.2004
(73) Proprietor: Kim, Yong-Gon, Seoul 137-851 (KR)
(72) Inventor: Kim, Yong-Gon, Seoul 137-851 (KR)
(74) Representative: Slingsby, Philip Roy
(86) International application number: PCT/KR2002/000793
(87) International publication number: WO 2002/087641

(56) References cited:
- EP-B- 0 376 448
- JP-A- 11 206 863
- KR-B- 128 110
- KR-B- 96 000 552
- US-A- 4 075 280
- US-A- 4 536 207

## Description

### TECHNICAL FIELD

The present invention relates to a multi-functional material emitting far-infrared ray in an aqueous phase and the use thereof. More specifically, the present invention relates to a multi-functional material emitting far-infrared ray in aqueous phase comprising extracts from shells of shellfishes and/or minerals, which can be used for various industrial purposes, and also to a deodorant produced therefrom.

### BACKGROUND ART

Far-infrared ray, which is a kind of electromagnetic wave, refers to a ray with a long wavelength among infrared rays (wavelength of 0.76-1000 µm), and generally indicates ray of a wavelength ranging from 4 to 1,000 µm although the precise limit of the wavelength range is not established. It exhibits a relatively strong thermal action compared to the visible ray, and its emission energy rapidly heats black bodies by the direct and instantaneous heat transfer. Thus, it is a useful microwave which is widely utilized owing to its excellent energy saving effect. Rays of such wavelength ranges are easily absorbed into a living body so that they act to stimulate and activate the molecules within the living body.

Materials which emit far infrared ray having the properties as described above are used in clothes and bedclothes, tableware and cooking devices, various building materials, a field of chemical industry, including drying agents for paints, dispersion aids and porous adsorbents, and fields of food processing and storage, as well as fields of agriculture, horticulture and stock raising. Also, these materials are used in a variety of health care devices, including thermo-therapeutic devices, such as saunas, thermal massage devices, mats and belts.

Prior art products using the far infrared ray-emitting materials have been produced by a method in which powdered bio-ceramics containing large amounts of the far infrared ray-emitting materials, including alumina and siliceous earth, are dispersed in an ammonia water, or cationic radicals are dispersed in resins, and the resulting composition is then coated on the surfaces of various products by adhesives.

However, these products using powders show insufficient emission of far infrared ray since the far infrared ray-emitting composition used as a raw material exists as a solid state. Also, in the case of textile products, they show insufficient emission of the far-infrared ray since the coating composition is not uniformly applied thereon, and in addition, they can show a far infrared ray-emitting effect only when heat of high temperature range, for example 200 to 500 C° is applied. For these reasons, it was very difficult to obtain an excellent far-infrared ray emission effect at room temperature or normal temperature.

A far infrared ray-emitting material that has been developed recently includes, for example, an aqueous(ionized) bio-ceramic for use in textiles disclosed in Korean Patent Publication No. 1996-15657 (November 20, 1996). This material is prepared by mixing sodium silicate, aluminum sodium, sodium oxide, sodium thiosulfate, germanium dioxide, purified dextrose and white sugar, dissolving the mixture into a solution and then ionizing the resulting solution.

However, such a ceramic product is disadvantageous in that the process for treating raw materials is complicated and also necessitates the use of strong acids which have an adverse effect against environment. In addition, this kind of product has a shortcoming that far infrared ray-emitting effect is weak. Therefore, an emission rate of far infrared ray can be increased only when it is subjected to high temperature treatment. For these reasons, most of them are insufficient for the desired functions.

Meanwhile, an offensive smell refers to an unpleasant and disgustful smell caused by irritating the sense of smell by hydrogen sulfide, mercaptans, amines and other gaseous irritating substances. In physical properties of the offensive smell, it is strong under the higher vapor pressure and under the greater solubility conditions. In addition, it is adsorptive and oxidative.

Methods for reducing the offensive smell include a ventilation and dilution method in which the offensive smell is controlled and diffused by a hood, a duct or a high chimney; an absorption method in which the offensive-smelling substances are passed through a washer so as to be absorbed into a washing solution; a condensation method in which the offensive-smelling substances are removed by condensation using a cooler; a combustion oxidation method in which the offensive-smelling substances are directly combusted with a flame of 600 to 800 °C; a catalytic oxidation method in which the offensive-smelling substances are treated using a catalyst, such as platinum; and a chemical oxidation method in which the offensive-smelling substances are chemically treated with O₃, KMnO₄ and chlorine compound, such as sodium hypochlorite. Also, as a method for disguising the offensive smell by spreading a component having a strong aromatic smell, a disguise method using perfumes, such as vanilin, rosin oil, benzyl acetate, is also employed. In addition, a neutralization method in which substances having other offensive smells are mixed to remove or reduce the offensive smell is often used.

Recently, a number of deodorants produced from various minerals as a raw material have been known. For example, Korean Patent Laid-Open Publication No. 2000-74767 (December 15, 2000) discloses a ceramic deodorant produced by a method in which various natural minerals, including serpentinite, amphibole and zirconium silicate are calcined at a high temperature of 1,200 to 1,300 °C, and the resulting material is then finely powdered, formed, dried and calcined. Korean Patent Laid-Open Publication No. 2000-74767 discloses a deodorant based on bio-stone(macksum-stone). However, the prior art production of the mineral-based deodorants requires a calcination step at high temperature in order to dissolve the minerals. In this case, deodorizing performance, such as intrinsic absorbing capability of the minerals, is reduced. Therefore, it is desired to develop a process for endowing the deodorants with the intrinsic properties of the minerals to improve the deodorizing performance.

### DISCLOSURE OF THE INVENTION

Accordingly, the present inventor has been intensively studied in order to improve the above-described drawbacks involved in the far infrared ray-emitting materials and to develop a far infrared ray-emitting material which has an excellent emission rate or strength of far infrared ray and can thus be used in various purposes. As a result, the inventor has found that an aqueous far infrared ray-emitting material produced by treating various minerals or shells of shellfishes by a method as will be described below and adding catalysts for activation, etc. to the treated materials has various biochemical functions in addition to a strong far infrared ray-emitting function and found that the deodorant produced therefrom has an excellent deodorizing capability against both the basic and acidic offensive-smelling substances and completed the present invention.

It is therefore an object of the present invention to provide an environment-friendly, odorless, colorless, and nontoxic far infrared ray-emitting liquid material which has various biochemical functions.

It is another object of the present invention to provide a method for producing the far infrared ray-emitting material.

It is a further object of the present invention to provide use of the far infrared ray-emitting material as a deodorant.

### BRIEF DESCRIPTION OF THE DRAWINGS

Fig. 1 is a graph showing far infrared ray emission strength measured on a plywood coated with an aqueous far infrared ray-emitting material of the present invention; and
Fig. 2 is a graph showing deodorizing capabilities of the deodorant prepared from the aqueous far infrared ray-emitting material of the present invention against various offensive-smelling substances.

### BEST MODE FOR CARRYING OUT THE INVENTION

The present invention, in an aspect, provides an aqueous environment-friendly, odorless, and nontoxic far infrared ray-emitting material having various biochemical functions, and also a method for preparing the same.

The aqueous far infrared ray-emitting material according to the present invention is a nontoxic strong alkaline material prepared by adding metal ions to aqueous extracts of natural minerals and shells of shellfishes. Since this material has various functionalities and emits far infrared ray at room temperature, it is a super-functional material in which various chemical changes can occur by minimum activation energy.

Agricultural products or living bodies (human and animal) show vigorous activity at room temperature or normal temperature. However, most of the prior bio-ceramic products (powders) emit far infrared ray at high temperature (50 to 300 °C). For this reason, it was impossible to expect a far infrared ray emission effect of the prior art products in these agricultural products or living bodies. However, it is possible by the invention to exhibit the far infrared ray emission effect even at room temperature or normal temperature (20 to 30 °C) by liquefying the natural minerals or the shells of shellfishes, and furthermore the far infrared ray emitting material of the invention was also contemplated as to the easy mixing with water by liquefying the far infrared ray-emitting material, and the storage stability, including prevention of putrefaction of water by activating water molecules.

The aqueous far infrared ray-emitting material according to the present invention can be prepared by a method comprising the steps of:
(a) mixing oyster shells, ferrous salts and/or ferric salts with aqueous alkaline solution and heating the mixture with stirring to produce an oyster shell-dissolved solution;
(b) dissolving zeolite in aqueous alkaline solution to produce a zeolite-dissolved solution;
(c) dissolving mackban-stone(quartz porphyry) in aqueous alkaline solution with heating and stirring to produce a mackban-stone-dissolved solution;
(d) mixing the thus produced solutions, sodium silicate, borax and potassium citrate and aging the mixture with heating and stirring to produce a solution of far infrared ray-emitting material; and
(e) passing the solution from the step (d) through magnetic materials to produce a magnetized aqueous far infrared ray-emitting material.

Hereinafter, the aqueous far infrared ray-emitting multifunctional material of the present invention and the method for preparing the same will be described in more detail.

As used herein, the term "an aqueous far infrared ray-emitting multifunctional material" is meant by a solution with various biochemical functions which emits a far infrared ray at room temperature in a liquid state and thus can be used in industrial applications.

In the specification and claims, various ratios at which raw materials, etc., are mixed, are for convenience indicated as values based on millimeter (ml) for liquid, and as values based on gram (g) for solid.

Far infrared ray-emitting components known in the art include sodium silicate, sodium aluminate, sodium thiosulfate, germanium dioxide, zinc oxide, potassium oxide, boron oxide, magnesium oxide, aluminum oxide (Al₂O₃), silica (SiO₂), sodium oxide, manganese oxide (MnO₂), copper oxide (CuO), iron oxide, zirconium oxide (ZrO₂), cobalt oxide, nickel oxide, chromium oxide, titanium oxide (TiO₂), molybdenum oxide, carbon-based materials, barium oxide, lithium oxide, ferric oxide (Fe₂O₃), clay, talc, loess and the like. These components are often found in various clays or minerals.

The far infrared ray-emitting material according to the present invention can be prepared by dissolving raw materials such as various minerals which contain the above components in a large amount, preferably silicate-rich minerals, for example, zeolite, mackban-stone and clay, or shells of shellfishes, preferably oyster shell in a solvent containing an aqueous alkaline solution, and then subjecting the resulting solution to physical and chemical treatment processes.

The shells of shellfishes contain a large amount of far infrared ray-emitting materials, such as calcium oxide (CaO) and magnesium oxide (MgO), and are strong alkali. Among the shells of shellfishes, oyster shell can preferably be used in the present invention since it is easily available and thus there is no burden for raw material costs. Collected oyster shells are thoroughly washed with an automatic washer to remove foreign substances including sand and filth, and dried. Then, the dried shells are powdered in a crusher and used as a raw material for the far infrared ray-emitting material. Iron salts, preferably ferrous salts and/or ferric salts are added to the powdered oyster shells, and then aged under stirring, using an aqueous alkaline solution as a solvent, at a desired temperature, for example, 100 to 150 °C, preferably about 100 °C or below, and more preferably 75 to 85 °C, for a suitable period of time, for example, 1 to 24 hours, preferably 1 to 7 hours, and more preferably about 3 hours to give an oyster shell-dissolved solution. It is preferred that the alkaline solution is a strong alkaline solution having a high pH. Examples of this strong alkaline solution include aqueous sodium hydroxide solution, aqueous potassium hydroxide solution, aqueous sodium carbonate solution, aqueous potassium carbonate solution and a mixture thereof. It is preferred to use a mixture of aqueous potassium carbonate solution and aqueous potassium hydroxide.

The aqueous alkaline solution of the invention which is used in dissolving the oyster shells or the minerals comprises, for example, a mixture of distilled water and potassium hydroxide at the volumetric ratio of 1:0.5, and preferably 1:0.42, or a mixture of distilled water and potassium carbonate at the volumetric ratio of 1:0.5, and preferably 1:0.42, respectively or in a combination. In preparing the oyster shell-dissolved solution of the invention, the above alkaline stock solutions are preferably mixed at the ratio of 2:1.

The iron salt serves to stabilize the oyster shell-dissolved solution. Small amount of ferrous salts and/or ferric salts is preferably added to the oyster shell-dissolved solution. Examples of ferrous salts which can be used in the present invention include inorganic iron salts, such as for example, ferrous oxide, ferrous sulfate, ferrous chloride, ferrous nitrate, ferrous bromide, ferrous iodide, etc. and organic iron salts, such as for example, ferrous gallate, ferrous mallate, ferrous fumarate, etc. Examples of ferric salts which can be used in the present invention include ferric oxide, ferric chloride, ferric hydroxide, ferric nitrate and the like.

In preparing the oyster shell-dissolved solution, the mixing ratio of KOH solution : K₂CO₂ solution : oyster shells : ferrous salts : ferric salts is preferably 5 to 20 : 2.5 to 10 : 1 to 5 : 0.1 to 2 : 0.1 to 2, and more preferably 8 to 15 : 3 to 8 : 2 to 3 : 0.1 to 2 : 0.1 to 2, and most preferably 10 : 5 : 2.52 : 0.15 : 0.15, based on volume and weight. In the oyster shell solution as described above, the amount of iron salts is most important. If iron salts are contained at the amount of less than 0.1 parts by weight, the functions of the aqueous far infrared ray-emitting material, i.e., functions as a catalyst and a stabilizer, are deteriorated. If the iron salts are contained at the amount of more than 2 parts by weight, there are no advantages in the function and effect. According to the test results carried out by the present inventor, even if the oyster shell-dissolved solution is produced at the mixing ratio which is somewhat out of the mixing ranges as specified above, there is no significant problem except that the emission capability of far infrared ray is somewhat reduced. Therefore, the mixing ratio range described above is specified to provide the easier understanding of the present invention, and the present invention is thus not limited only to the above mixing ratio range. It will be understood that, where the oyster shell-dissolved solution prepared at a mixing ratio which is somewhat out of the range specified above exhibits the effect intended for the present invention, it is within the scope of the present invention.

In preparing the oyster shell-dissolved solution high temperature treatment may result in vaporization of ingredients including calcium compounds, and thus the far infrared ray-emitting material has no usefulness. Therefore, the mixture is preferably stirred at a low temperature of 150 °C or below so that intrinsic characteristics of the raw materials are not modified.

Additional components of the aqueous far infrared ray-emitting material of the invention comprise dissolved minerals including zeolite, mackban-stone and clay. Such minerals are treated in the similar manner as described above.

Zeolite, a porous material, has widely been used as an absorbent in the fields of chemical experiment or chemical industry, or as a hard water softener using ion exchange and the like. Also, it has been used along with microorganisms in removing an offensive smell from the excretions or other wastes. Zeolite is a kind of aluminosilicate mineral, and cavities at molecular level consisting of TO₄ (T denotes Si or Al that is a tetrahedral atom) in tetrahedron are three-dimensionally linked with each other to form passages at molecular level. Reversible adsorption/desorption phenomena of water molecules occur within the cavities of zeolite, and there are about 40 kinds of natural zeolite and about 130 kinds of synthetic zeolite. The zeolite which can be used in the present invention encompasses those are known in the art and has no specific limitation.

In order to obtain the zeolite-dissolved solution according to the present invention, zeolite is added to an aqueous stock solution of potassium hydroxide as described above and dissolved for a desired period of time, for example 3 hours, without any specific conditions to give the zeolite dissolved solution. If heat is applied in the process of preparing the zeolite-dissolved solution, there is a concern that zeolite become a slurry. Therefore, it is preferred that heat is not applied in this process. In order to prepare the zeolite-dissolved solution, the aqueous stock solution of potassium hydroxide is mixed with zeolite at the mixing ratio of preferably about 2.5 to 10 : 0.5 to 2, more preferably 5 : 1, and most preferably 5 : 0.84. This mixing ratio is set in order to provide the easier understanding of the present invention, and the present invention is not limited only to this range. It will be understood that, where the zeolite-dissolved solution prepared by a mixing ratio which is somewhat out of the range specified above exhibits the effect intended in the present invention, it is also within the scope of the present invention.

Mackban-stone(mineral stone, quartz porphyry) has a porous structure and is a yellowish white-colored mineral, the main component of which comprises silica, aluminum oxide, iron oxide, magnesium oxide and calcium oxide, and thus is also suitable as a material for the far infrared ray-emitting material.

The mackban-stone dissolved solution of the invention can be prepared by adding mackban-stone to the aqueous stock solution of potassium hydroxide and then treating the resulting mixture under the same condition as in the preparation of the oyster shell-dissolved solution. Namely, an aqueous alkaline solution containing mackban-stone is aged with stirring at a desired temperature, for example, 100 to 150 °C, preferably less than 100 °C, and more preferably 75 to 85 °C, for a suitable period of time, for example, 1 to 24 hours, preferably 1 to 7 hours, and more preferably about 3 hours to give the mackban-stone dissolved solution of the present invention.

In the preparation of the mackban-stone dissolved solution, the mixing ratio of potassium hydroxide solution : mackban-stone is preferably 2.5 to 10 : 0.5 to 2, more preferably 5 : 1, and most preferably 5 : 0.84, based on the weights. This mixing ratio is set in order to provide the easier understanding of the present invention, and the present invention is not limited only to this range. It will be understood that, where the mackban-stone dissolved solution prepared by a mixing ratio which is somewhat out of the range specified above exhibits the effect intended in the present invention, it is also within the scope of the present invention.

In addition to the above components, a clay-dissolved solution can also be used in the present invention since it contains silicon, aluminum, iron, magnesium, alkali metals, alkaline earth metals and the like. In addition, it is possible that various alkali dissolving solutions may be made by treating macksum-stone(bio-stone), serpentinite, amphibole, bentonite, diatomite, etc. with the aqueous alkaline solution of the invention and used as the mineral-dissolved solutions.

Each mineral solution prepared as described above may be separately used in the present invention since it individually can emit far infrared ray. However, it is more preferred that the far infrared ray-emitting material having various biochemical functions be prepared by mixing the above listed materials at a desired ratio.

The aqueous far infrared ray-emitting material according to the present invention can be produced by mixing the oyster shell-dissolved solution, the zeolite-dissolved solution, the mackban-stone-dissolved solution, a sodium silicate solution (Na₂O·nSi₂-xH₂O, water glass), borax (Na₂B₄O₇) and potassium citrate (K₂C₆H₅O₇), at a desired ratio, and then aging with stirring at a temperature range of room temperature to less than 100 °C, for a suitable period of time, for example, 10 minutes to 5 hours, preferably 10 minutes to 3 hours, and more preferably about 1 hour to give a mixture of far infrared ray-emitting materials. In the preparation of the aqueous far infrared ray-emitting material, it is preferred that temperature higher than 100 °C is not applied.

Also, an aqueous silver solution and germanium dioxide as minor components may be added to the above mineral-dissolved solutions.

In the preparation of the aqueous far infrared ray-emitting material, the mixing ratio of oyster shell dissolved solution : zeolite dissolved solution : mackban-stone dissolved solution : sodium silicate : borax : potassium silicate is preferably 10 to 40 : 2 to 10 : 5 to 20 : 5 to 20 : 1 to 5 : 1 to 5, more preferably 10 to 40 : 3 to 8 : 8 to 15 : 8 to 15 : 1 to 5 : 1 to 5, and most preferably 20 : 5 : 10 : 10 : 3 : 3, based on the weights. Of course, even if the above composition deviates the specified mixing ratio range, there is no significant problem except that the emission capability of far infrared ray is somewhat reduced. Therefore, the composition of the aqueous far infrared ray-emitting material according to the present invention is not limited only to the above range. It will be understood that, where the aqueous far infrared emitting material prepared by a mixing ratio which is somewhat out of the range specified above exhibits the desired product quality, it is also within the scope of the present invention.

If the aqueous mixture containing the above minerals is passed through a permanent magnet having a capacity of 15,000 to 30,000 G, for example, a device which generates a magnetic field by an ND magnet, the solution crossed perpendicularly to the magnet shows properties of magnetized water thereby giving a magnetized aqueous far infrared ray-emitting of the invention.

The aqueous far infrared ray-emitting material thus prepared contains potassium (0.17%), sodium (0.05%), calcium (0.001%) and silicon (0.0001%), as its major elemental components. Thus, it contains alkaline earth metals as the major components and various minerals as the minor components so that it can perform intrinsic functions of far infrared ray-emitting ceramics or ores with very low energy even at room temperature and in a liquid state.

The aqueous far infrared ray-emitting material containing iron salts which was 100-fold diluted with distilled water has properties of magnetized water and has a boiling point of 95 °C which is lower than that of normal water. And, as the liquid in which the coupling unit of water molecules is small, it represents a strong alkali (pH 11-13, 20 °C), and a specific gravity of 1.010 (20/4°C) and drying loss of 98.0.

Contents of heavy metals and environmentally harmful substances of the far infrared ray-emitting material of the invention are shown in Table 1 below.

**Table 1:**

| Test items | Unit | Results | Test method |
|---|---|---|---|
| Cu | ppm | 0.6 | I.C.P. analysis |
| Cd | ppm | not detected | I.C.P. analysis |
| Pb | ppm | not detected | I.C.P. analysis |
| As | ppm | not detected | I.C.P. analysis |
| Hg | ppm | not detected | I.C.P. analysis |
| CN | ppm | not detected | KS M 0111-98 |
| Cr | ppm | 0.1 | I.C.P. analysis |
| Zn | ppm | 0.3 | I.C.P. analysis |
| Mn | ppm | 0.2 | I.C.P. analysis |
| Fe | ppm | 2.7 | I.C.P. analysis |
| Se | ppm | not detected | I.C.P. analysis |
| Al | ppm | 1.8 | I.C.P. analysis |
| Benzene | mg/l | not detected | GC(FID) |
| Toluene | mg/l | not detected | GC(FID) |
| Phenol | mg/l | not detected | Water contamination test method |

From the results shown in Table 1, it can be found that the aqueous far infrared ray-emitting material according to the present invention contains little or no heavy metals or environmentally harmful substances, and thus it has no adverse effect on human body, animals and environment.

The above stock solution can be diluted up to 200 times depending upon the desired applications.

The present invention in an additional aspect provides the use of the aqueous far infrared ray-emitting material as a deodorant.

The deodorant of the present invention is prepared by mixing the aqueous far infrared ray-emitting material with water, potassium persulfate, sodium hypochlorite, etc. and then stirring the resulting mixture for about 2 hours.

As the additional components, ascorbic acid, sugar (refined sugar or white sugar), potassium permanganate, sodium permanganate, germanium dioxide, magnesium sulfate and the like may be added.

The mixing ratio of the far infrared ray-emitting material stock solution : water : potassium persulfate : sodium hypochlolite is preferably 3 to 40 : 20 to 100 : 0.5 to 5 : 0.5 to 5, and most preferably 10 : 100 : 1.5 : 1, based on the weights. Among the above components, the content of potassium persulfate is important. Where the content of potassium persulfate is less than 0.5, it is disadvantageous in that a deodorizing effect is reduced. Where the content of potassium persulfate is more than 5, it is uneconomical since there is no increase in deodorizing effect. This deodorant solution can be used by diluting up to 200 times depending upon the desired applications. The additional components as described above may be used at a similar amount to the range of potassium persulfate.

The deodorant according to the present invention contains no heavy metals or environmentally harmful substances, and is nontoxic, non-irritative, and thus environment-friendly. Also, since it has an excellent deodorizing capability against both the acidic and the basic offensive-smelling substances, it is used for the surrounding environment as well as animals.

### Examples

The present invention will hereinafter be described in further detail by examples. It should however be borne in mind that the present invention is not limited to or by the examples.

### Example 1: Preparation of mineral-dissolved solutions

Collected oyster shells were washed in an automatic washer to remove sand, filth and the like. The washed shells were dried and powdered in a crusher. This pre-treated oyster shells were mixed with ferrous sulfate, ferric oxide (Fe₂O₃) and a solvent at the mixing ratio shown in Table 2 below, and then the mixture was aged with stirring at 80 °C for 3 hours to give an oyster shell-dissolved solution. Similarly, a zeolite (Clinoptilolite-(Na₂, Ca) Al₂Si₇O₁₈ · 6H₂O); Mordenite-Na₂ (AlSi₅)₁₂)₂·7H₂O may be alternatively used) dissolved solution and a mackban-stone dissolved solution were prepared.

**Table 2:**

| | Components | Examples | | | | |
|---|---|---|---|---|---|---|
| | | 1a | 1b | 1c | 1d | 1e |
| Oyster shell dissolved solution (80 °C, stirring for 3 hrs) | Potassium hydroxide solution* | 500 ml | 750 ml | 1000 ml | 1500 ml | 2000 ml |
| | Potassium carbonate** | 1000 ml | 750 ml | 500 ml | 500 ml | 250 ml |
| | Oyster shells | 120 g | 175 g | 252 g | 425 g | 500 g |
| | Ferrous sulfate | 5 g | - | - | 10g | 15 g |
| | Ferric oxide | 20 g | 15 g | 15 g | 5 g | 5 g |
| | Ferric chloride | - | 10 g | 15 g | 15 g | 20 g |
| Zeolite dissolved solution (stirring for 30 minute) | Potassium hydroxide solution* | 125 ml | 175 ml | 250 ml | 500 ml | 1000 ml |
| | Zeolite | 200 g | 100 g | 42 g | 35 g | 25 g |
| Mackbanstone dissolved solution (80 °C, stirring for 3 hrs) | Potassium hydroxide solution* | 200 ml | 300 ml | 500 ml | 750 ml | 1000 ml |
| | Mackban-stone | 400 g | 200 g | 84 g | 70 ml | 50 g |

| | | | | | | |
|---|---|---|---|---|---|---|
| * : prepared by mixing 1000 ml of distilled water and 420 g of potassium hydroxide | | | | | | |
| ** : prepared by mixing 1000 ml of distilled water and 420 g of potassium carbonate | | | | | | |

### Example 2: Preparation of aqueous far infrared ray-emitting material

The respective dissolved solutions prepared from Example 1, a sodium silicate solution (Na₂O · nSiO₂-xH₂O), borax (Na₂B₄O₇) and potassium citrate (K₂C₆H₅O₇) were mixed at the amount as shown in Table 3 below. The mixture was aged with stirring at 80 °C for about 30 minutes to give the aqueous far infrared ray-emitting material of the present invention. The far infrared ray-emitting solution containing the minerals was passed through a device generating a magnetic field by an ND magnet having a capacity of 15,000 to 30,000 G (trade name: NO-FE-B, manufacturer: Seouljasuk, Korea) to give a magnetized far infrared ray-emitting material.

**Table 3:**

| Component | Example | | | | |
|---|---|---|---|---|---|
| | 2a | 2b | 2c | 2d | 2f |
| Oyster shell-dissolved solution | 100 ml | 150 ml | 200 ml | 300 ml | 400 ml |
| Zeolite-dissoloved solution | 100 ml | 75 ml | 50 ml | 30 ml | 20 ml |
| Mackban-stone dissolved solution | 50 ml | 75 ml | 100 ml | 150 ml | 200 ml |
| Sodium silicate solution | 200 ml | 150ml | 100 ml | 75 ml | 50 ml |
| Borax | 10 g | 20 g | 30 g | 40 g | 50 g |
| Potassium silicate | 50 g | 40 g | 30 g | 20 g | 10 g |
| Germanium dioxide | - | 2 g | - | - | - |
| Aqueous silver solution* | - | - | - | 5 ml | - |

| | | | | | |
|---|---|---|---|---|---|
| * aqueous silver solution: electrolyzed electrolytic water-silver solution | | | | | |

### Example 3: Preparation of deodorant

The aqueous far infrared ray-emitting material of Example 2c was mixed with the components as shown in Table 4, and then stirred for 2 hours at room temperature (28 to 30 °C) to give a deodorant. The thus prepared stock solution can be diluted up to 20 times depending on the desired applications.

**Table 4:**

| Component | Example | | | | |
|---|---|---|---|---|---|
| | 3a | 3b | 3c | 3d | 3e |
| Far infrared ray-emitting stock solution | 10 ml | 25 ml | 50 ml | 75 ml | 100 ml |
| Sodium hypochlorite | 10 ml | 7.5 ml | 5 ml | 2.5 ml | 1 ml |
| Ascorbic acid | 5 g | - | - | - | - |
| Sugar (refined sugar) | 50 g | - | 100 g | 150 g | 200 g |
| Potassium persulfate | 1g | 5 g | 7.5 g | 10 g | 15 g |
| Potassium permanganate | - | 2 g | - | - | - |
| Sodium permanganate | - | - | - | 5 g | - |
| Germanium dioxide | 15 g | - | - | - | - |
| Magnesium sulfate | - | - | - | - | 10 g |
| Distilled water | 500 ml | 500 ml | 500 ml | 500 ml | 500 ml |

### Test example 1: Measurement of far infrared ray emission capability

A far infrared ray-emitting material in a liquid state is technically difficult to measure the emission rate and the emission power of far infrared ray. Thus, the test for far infrared ray emission capability of the present invention (Example 3c) was carried out in an indirect manner by coating plywood with the aqueous material of the invention and then tested with an IR-spectroscope at Far Infrared Ray Application and Evaluation Center, Korea Institute of Construction and Material (KICM). As a result, the control plywood exhibited an emission rate of 0.904 and an emission energy of 3.65 x 10²(w/m²) at 40 °C and a wavelength range of 5 to 20 µm, whereas the aqueous far infrared ray-emitting material of the invention exhibited an emission rate of 0.909 and an emission energy of 3.66 x 10²(w/m²) at the same condition.

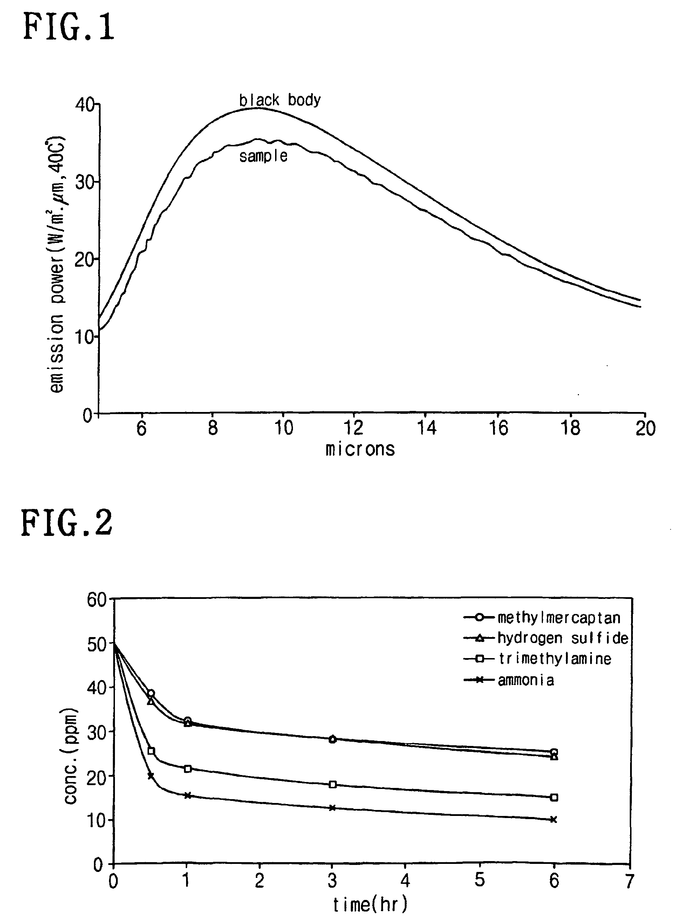

Fig. 1 is a graph showing the emission strength measured on the plywood coated with the aqueous far infrared ray-emitting material. As shown in Fig. 1, the aqueous material of the invention was excellent in that the emission strength is nearly similar to that of a black body showing the maximum emission rate of far infrared ray at a wavelength of more than 5.0 µm.

According to the test results, it was confirmed that the aqueous far infrared ray-emitting material of the present invention can emit far infrared ray at room temperature although the test was carried out indirect manner in which the emission was not directly tested in a liquid state at 40 °C. Also, it is obvious that if test temperature is increased the far infrared ray emission rate will increase since metals are changed into metal oxides.

### Test Example 2: Tests on the deodorizing capability

Deodorant (Example 3c, 10-fold diluted) prepared from the aqueous far infrared ray-emitting material of the present invention was tested for deodorizing capability with trimethylamine, and hydrogen sulfide and methyl mercaptane as acidic offensive-smelling substances, by requesting at the Korea Testing and Research Institute for Chemical Industry. As the tester, Model 801 (GASTEC Co., Japan) was used. Briefly, ammonia, an offensive-smelling source was introduced into a device for deodorization test (40 cm x 40 cm x 60 cm) and adjusted to its concentration at 50 ppm with the GASTEC. Also, trimethylamine, hydrogen sulfide and methylmercaptan were similarly introduced into the respective vessels for deodorization test and adjusted to their concentrations at 50 ppm. Then, 20 ml of the deodorant solution to be tested was injected with an injector into each of the devices which had been adjusted to the initial concentration of the offensive-smelling substances. Concentrations of the offensive-smelling substances were measured with the tester after 30 minutes, 1 hour, 3 hours and 6 hours. The experimental conditions were as follows: indoor temperature of 22 °C ± 5°C, and relative humidity of 43% ± 5%. Results are shown in Fig. 2.

According to the results shown in Fig. 2, the basic offensive-smelling substances, ammonia and trimethylamine were removed after 6 hours up to 81.0% and 71.0%, respectively, relative to the initial concentration. The acidic offensive-smelling substances, hydrogen sulfide and methylmercaptan were removed after 6 hours up to 53.0% and 51 %, respectively, relative to the initial concentration. It can be seen from the above results that the deodorant of the present invention has an excellent deodorizing capability against all the basic and acidic offensive-smelling substances.

### Test Example 3: Acute toxicity test

The deodorant according to Example 3c was subjected to an acute toxicity test using test animals, and observed as to whether it has toxicity or not. The test animals were sixty SPF SD series rats (purchased from Somangchuksan, Korea) consisting of 4-5 weeks old male (body weight of 105 ± 4 g) and female (body weight of 95 ± 3 g). The test was carried out using the deodorant solution and distilled water as a negative control. The rats were acclimated for about one week in a laboratory breeding box with the following conditions: temperature of 22 ± 2 °C, relative humidity of 53 ± 2%, light/dark cycle of lighting at 09:00-putting out lights at 18:00 using illumination of fluorescent lamp, and illumination intensity of 150-300 Lux. Among the acclimated animals, only healthy animals were selected and divided into the respective groups according to the body weight. The deodorant of the present invention was forcibly orally administered to the selected animals at the amount of 20 ml/kg, one time each day for 14 days. Then, the animals were checked for the changes in general conditions, toxic signs, appearances, autonomic nervous system, changes in body weight and the death of animals.

According to the test results, the test animals exhibited a change within 5% in body weights for the test period, but there was no significance. Also, any dead animal was not observed even if the amount of the sample was set to the concentration of 20 ml/kg which is the maximum amount to be administered in a day. Therefore, it was evaluated that LD₅₀ of the deodorant of the invention is 20 ml/kg B.W. or above. In addition, since any specific signs or lesions were not observed upon necropsy. In view of the above results, the above deodorant was proved to be nontoxic.

### Test Example 4: Ocular mucosa irritation test

An ocular mucosa irritation test was carried out in order to examine the irritation level against test animals of the deodorant according to Example 3c.

Nine New Zealand white rabbits (body weight of 2.5-2.8 kg, 4 month old, purchased from Somangchuksan, Korea) were used as the test animals. First, the test animals were acclimated in an animal chamber for 2 weeks while observing general conditions. Among the acclimated animals, only healthy animals were used in the test. At about 24 hours before starting of the test, the rabbits were examined for pathological signs of cornea, conjunctiva and iris of the right and left eyeballs. Among the examined rabbits, rabbits with the healthy both eyes were selected for the test. The test material was administered dropwise to an eye of the rabbits at the amount of 0.1 ml per rabbit. Among the rabbits administered with the test material, three rabbits were eye-washed for their both eyes with a sterile saline solution for 1 minute at 20-30 seconds after administration of the test material, and the remaining six rabbits were not eye-washed. At 1, 2, 3, 4 and 7 days after administration of the test material, and then with 3 days interval over 13 days or more, eye conditions of the rabbits were observed compared with another eye not-administered with the test material as a control group. The rabbits were observed for general condition, and ingestion of feedstuff and water, etc.

Marking of ocular lesions and evaluation of irritation were conducted in accordance with "toxicity test standards for drug, etc." according to Notice of Korea Food and Drug Administration No. 1999-61, and "Standard Operation Guidebook" published by the Korea Food and Drug Administration. Irritation level was determined on the basis of the above ocular lesion marking table and the ocular mucosa irritation evaluation table. Specifically, marks of cornea (maximum value: 80), iris (maximum value: 10) and conjunctiva (maximum value: 20) were calculated at each observation using the scored ocular mucosa irritation and the total marks of the respective animals were obtained. The mean ocular irritation index (M.O.I). M.O.I. is defined by dividing the sum of the total marks of the respective animals by the number of animals. During the observation, strength of ocular mucosa irritability was evaluated based on the acute ocular irritation index (A.O.I) which is the maximum value of M.O.I.

According to the test results, any pathological signs caused by the irritation after administration of the test material were not observed, and dead animal was not observed. Furthermore, any clinical signs in cornea, iris and conjunctiva were not observed at 1, 2, 3, 4 and 7 days after administration of the test material. Therefore, it was confirmed that A.O.I. is "0" and thus the test material was a non-irritative material.

### Test Example 5: Skin irritation test

Skin irritation test was carried out in order to examine the irritation level on the test animals of the deodorant according to Example 3c. Six New Zealand white rabbits (body weight of 2.5-2.8 kg, 3-4 month old (purchased from Somangchuksan, Korea) were used as the test animals. First, the test animals were acclimated in an animal chamber for 1 week while checking general conditions. Among the acclimated animals, only healthy animals were used in the test. At 24 hours before application of the test material, the rabbits were subjected to fur removal, and then they were divided into the treating group and the control group.

0.5 Ml of the test material per animal was applied one time to the treating group while the same amount of a sterile saline solution was applied to the control group. After application of the test material, the applied sites were covered with a thin paper of solid material, and fixed with a tape, and this state was then maintained for a desired time. After lapse of the application time, each of the applied sites was gently washed with a sterile saline solution. The animals were observed every day for their appearance, consumption states of feedstuff and water, and clinical signs for one week. At 7 days after application, the rabbits were injected with a local anesthetic (lidocaine, Kwangmyung Pharmaceutical Co., Korea) via ear veins, and euthanized. After necropsy, the rabbits were observed with the naked eye for abnormal conditions.

Skin response was evaluated at 24 hours and 48 hours using "toxicity test standards for drug, etc." according to the Notice of Korea Food and Drug Administration No. 999-61. Also, the skin irritation level was determined according to the commonly used Primary Irritation Index (P.I.I.) by Draize.

From the test results, irritation, including erythema, eschar formation and edema, was not recognized at the test material-applied sites. Also, the primary irritation index of Draize was found to be "0".

From the test results as described above, it was confirmed that the deodorant according to the present invention has no toxicity or irritability on the test animals. Therefore, it is obvious that the far infrared ray-emitting material, which is the starting material of the deodorant, also has no adverse effect on surroundings or animals.

### INDUSTRIAL APPLICABILITY

As apparent from the test examples described above, the aqueous far infrared ray-emitting material of the present invention is environment-friendly, odorless, colorless and nontoxic. In addition, this liquid material can emit far infrared ray at room temperature, and can trigger various chemical changes by minimum activation energy. For this reason, it exhibits an energy saving effect, and also can accelerate metabolism of animals and plants, or other biological functions via activation of water molecules. In particular, since the aqueous far infrared ray-emitting material of the present invention contains ferrous salts or ferric salts at a very small amount, and is similar to an oxygen rich non-ionized water and thus has a geometrical structure close to biological water, it has a very stable structure which is not involved in oxidation-reduction reaction in an aqueous phase. Also, it has high intracellular permeability so that it can maximize intracellular metabolism with relatively small energy. Moreover, it is effective in preventing propagation of microorganisms and has excellent storage stability, and it thus can also be used as bactericides and preservatives. The far infrared ray-emitting material of the present invention shows a synergistic effect for desired applications when it is mixed with various functional materials in a liquid state. It can be used alone in a certain application. In addition, the deodorant prepared from the aqueous material of the present invention has an excellent deodorizing effect against both the acidic and basic offensive-smelling substances without adverse effect, such as toxicity, the environment or animals.

## Claims

1. A method for preparing an aqueous deodorant, comprising the steps of:
(a) mixing oyster shells, iron salts with an aqueous alkaline solution and heating the mixture with stirring to produce an oyster shell-dissolved solution;
(b) dissolving zeolite in an aqueous alkaline solution to produce a zeolite-dissolved solution;
(c) dissolving quartz porphyry in an aqueous alkaline solution with heating and stirring to produce a quartz porphyry-dissolved solution;
(d) mixing the thus produced solutions, sodium silicate, borax and potassium citrate and aging the mixture with heating and stirring to produce a solution of far infrared ray-emitting material;
(e) passing the solution from step (d) through a permanent magnet to produce a magnetized aqueous far infrared ray-emitting material; and
(f) mixing the aqueous far infrared ray-emitting material with water, potassium persulfate and sodium hypochlorite with stirring.

2. The method of Claim 1, wherein the mixing ration of the aqueous far infrared ray-emitting material water : potassium persulfate : sodium hydrochlorite is 3 to 40 : 20 to 100 : 0.5 to 5:0.5 to 5.

3. The method of Claim 1, wherein the mixing ratio of aqueous far infrared ray emitting material : water : potassium persulfate : sodium hydrochlorite is in the range of 10 : 100 : 1.5 : 1.

4. The method of claim 1, wherein the deodorant further comprises one or more components selected from the group consisting of ascorbic acid, refined sugar, germanium dioxide, potassium permanganate, sodium permanganate and magnesium sulphate.

5. An aqueous deodorant prepared according to any of claims 1 to 4.

## Revendications

1. Procédé de préparation d'un désodorisant aqueux, comprenant les étapes consistant à :
(a) mélanger des coquilles d'huître, des sels de fer avec une solution alcaline aqueuse et chauffer le mélange sous agitation pour produire une solution de coquilles d'huître dissoutes ;
(b) dissoudre de la zéolite dans une solution alcaline aqueuse pour produire une solution de zéolite dissoute ;
(c) dissoudre du porphyre de quartz dans une solution alcaline aqueuse sous chauffage et agitation pour produire une solution de porphyre de quartz dissous ;
(d) mélanger les solutions ainsi produites, du silicate de sodium, du borax et du citrate de potassium et faire vieillir le mélange sous chauffage et agitation pour produire une solution de matière émettant un rayonnement infrarouge lointain ;
(e) faire passer la solution de l'étape (d) à travers un aimant permanent pour produire une matière émettant un rayonnement infrarouge lointain aqueuse magnétisée ; et
(f) mélanger la matière émettant un rayonnement infrarouge lointain aqueuse avec de l'eau, du persulfate de potassium et de l'hypochlorite de sodium en agitant.

2. Procédé selon la revendication 1, dans lequel le rapport de mélange de matière émettant un rayonnement infrarouge lointain aqueuse : eau : persulfate de potassium : hypochlorite de sodium est de 3 à 40 : 20 à 100 : 0, 5 à 5 : 0,5 à 5.

3. Procédé selon la revendication 1, dans lequel le rapport de mélange de matière émettant un rayonnement infrarouge lointain aqueuse : eau : persulfate de potassium : hypochlorite de sodium est de l'ordre de 10 : 100 : 1,5 : 1.

4. Procédé selon la revendication 1, dans lequel le désodorisant comprend de plus un ou plusieurs composants choisis dans le groupe formé par l'acide ascorbique, le sucre raffiné, le bioxyde de germanium, le permanganate de potassium, le permanganate de sodium et le sulfate de magnésium.

5. Désodorisant aqueux préparé selon l'une quelconque des revendications 1 à 4.

## Patentansprüche

1. Verfahren zur Herstellung eines wässrigen Deodorants, umfassend die Schritte:
(a) Mischen von Austernschalen, Eisensalzen mit einer wässrigen alkalischen Lösung und Erhitzen der Mischung unter Rühren, um eine Lösung von gelösten Austernschalen herzustellen;
(b) Auflösen von Zeolith in einer wässrigen alkalischen Lösung, um eine Lösung von gelöstem Zeolith herzustellen;
(c) Auflösen von Quarzporphyr in einer wässrigen alkalischen Lösung unter Erhitzen und Rühren, um eine Lösung von gelöstem Quarzporphyr herzustellen;
(d) Mischen der so hergestellten Lösungen, Natriumsilikat, Borax und Kaliumzitrat und Altern der Mischung unter Erhitzen und Rühren, um eine Lösung von Ferninfrarotstrahlung emittierendem Material herzustellen;
(e) Durchführen der Lösung aus Schritt (d) durch einen Permanentmagneten, um ein magnetisiertes, wässriges, Ferninfrarotstrahlung emittierendes Material herzustellen; und
(f) Mischen des wässrigen, Ferninfrarotstrahlung emittierenden Materials mit Wasser, Kaliumpersulfat und Natriumhypochlorit unter Rühren.

2. Verfahren nach Anspruch 1, wobei das Mischungsverhältnis von wässrigem, Ferninfrarotstrahlung emittierendem Material:Wasser:Kaliumpersulfat:Natriumhypochlorit 3-40:20-100:05,-5:0,5-5 beträgt.

3. Verfahren nach Anspruch 1, wobei das Mischungsverhältnis von wässrigem, Ferninfrarotstrahlung emittierendem Material:Wasser:Kaliumpersulfat:Natriumhydrochlorit im Bereich von 10:100:1,5:1 liegt.

4. Verfahren nach Anspruch 1, wobei das Deodorant ferner eine oder mehr Komponenten umfasst, die ausgewählt sind aus der Gruppe bestehend aus Ascorbinsäure, raffiniertem Zucker, Germaniumdioxid, Kaliumpermanganat, Natriumpermanganat und Magnesiumsulfat.

5. Wässriges Deodorant, hergestellt gemäß einem der Ansprüche 1 bis 4.
